# EUROPEAN PATENT APPLICATION

(11) **EP 3 671 700 A1**
(43) Date of publication of application: **24.06.2020**
(21) Application number: 19169496.7
(22) Date of filing: 16.04.2019
(51) Int. Cl.: G09B 19/00, A61B 5/00

(54) **A METHOD OF PERFORMING SENSOR PLACEMENT ERROR DETECTION AND CORRECTION AND SYSTEM THERETO**

(30) Priority: 19.12.2018 PT 2018115220
(71) Applicant: SWORD Health S.A., 4000-437 Porto (PT)
(72) Inventor: Branquinho, André Gomes, 3810-056 Aveiro (PT); Ferreira Matos, Ana Clara, 4000-437 Porto (PT); Pinto Coelho, Luís Ungaro, 4000-437 Porto (PT); Ferro Bento, Virgílio António, 4000-437 Porto (PT)
(74) Representative: Balder IP Law, S.L.

(57) **Abstract**

The present invention relates to a method of performing sensor placement error detection and correction for a system (10) for tracking human motion comprising
- at least one sensor unit (12,14),
- at least one sensor holder (16,18),
wherein the method comprises at least the following steps forming a method routine:
- Analyzing, whether there is at least one unmet condition, indicating an incorrect sensor unit placement;
- In case of detecting such at least one unmet condition preparing a list of potential reasons for the incorrect sensor unit placement;
- Remove the possible reasons for incorrect sensor unit placement already presented to the user;
- Select the one of the reasons and present it to a user.

Furthermore, the above-mentioned invention relates to a system (10) for tracking human motion.

## Description

The present invention relates to a method of performing sensor placement error detection and correction and system thereto.

During rehabilitation, one of the challenges is to control rehabilitation exercises.

From EP 2 750 120 A1 an exercise information display system and exercise information display method is already known. The system includes a sensor device, which obtains data associated with a motion status of a human body during an exercise, a data processing device which generates plural types of exercise information based on the data obtained by the sensor device and a viewing device which displays from among the plural types of exercise information at least first information indicating a posture of the human body during the exercise and second information associated with the first information in the display format, where the first information and a second information are displayed in connection with each other.

A system capable of tracking human motion requires the user to attach a set of sensors to a set of straps he is wearing. Each of those sensors must be placed on anatomical landmarks depending upon the limb whose motion is to be tracked. The same system also requires the user to attach the sensors to a sensor station for the purpose of charging them as well as performing an inspection thereof. As such, the following is required for using this system:
- Each sensor must be matched to the corresponding strap before the motion tracking procedure, as the system assumes an anatomical landmark for each sensor - this may be accomplished by using a color code that matches the sensor to the strap of the same color or, alternatively, by the means of calibration movements before usage.
- The sensors must be kept correctly attached to the strap throughout the motion tracking procedure.
- The sensors must be correctly attached to the sensor station for the charging and inspection procedures.

Due to these requirements and the fact that it is not clear for the user what he must do at a given point while using the system, the following problems arise:
- The user does not place the sensors in the straps because he does not understand he must do so.
- The user misplaces the sensors by having them incorrectly attached to the straps.
- The user misplaces the sensors by switching positions between two sensors since this match is cumbersome and prone to failure.
- The sensors may detach during the movement without the user realizing.
- The user does not place the sensors in the station because he does not understand he must do so.

These events result in a corruption of the motion tracking procedure, an invalid sensor inspection and a lack of battery of the sensors. In sum, using the system correctly requires great understanding of its requirements (which impairs the user experience) and failing to do so has severe consequences.

It is therefore an option of the present invention to provide a system for tracking human motion which provides an enhanced solution for correct sensor placement and avoiding misplacement of the sensors.

This object is solved according to present invention by a method of performing sensor placement error detection and correction.

Accordingly, the method of performing sensor placement error detection and correction for a system for tracking human motion is performed such that the method comprises at least the following steps forming a method routine:
- Analyzing, whether there is at least one unmet condition, indicating an incorrect sensor placement;
- In case of detecting such at least one unmet condition preparing a list of potential reasons for the incorrect sensor placement;
- Remove the possible reasons for incorrect sensor placement already presented to the user;
- Select the one of the reasons and present it to a user.

The system comprises at least a sensor unit and at least a sensor holder.

The invention is based on the basic idea that incorrect sensor placement can be checked in a very easy and at the same time effective way. In particular, the inventors have found that incorrect sensor placement in most cases has only a limited amount of reasons and within this list of reasons, there are reasons with higher numbers than others. It is therefore possible to prepare a list of potential reasons for the incorrect sensor placement and to present these reasons the user, then check whether the sensor is now correctly placed and confirm with user feedback, whether the system is now in a condition, where one or all sensors or at least one sensor is now put into correct condition.

Moreover, the reasons are hierarchically ranked. In particular, it would be possible to use the information, which reasons have a higher likelihood and to put them in the ranking on top whereas other reasons, with less likelihood are put in lower ranking positions. By this, the advantages achieved that with a higher likelihood only a few check routine steps must be done.

Furthermore, it is possible that it is checked, which reasons have already been presented. Thus, iterations must not be done and can be avoided.

Additionally, it can be checked, whether still reasons are left. By this step, it can be avoided that after complete check-up the system starts again. Also, if it is found that no reasons are left, then a general remark regarding the manual can be provided in this plate.

In case that no reasons are left, the method routine can be ended. Then, the method routine can be stopped.

If, however, further reasons are left, then the hierarchically top ranked reason may be selected and presented to the user. With the hierarchically top ranked reason preferably the reason is meant, which is within the ranking the next top ranked reason in the ranking, considering the already shown ranked reasons, which are not under consideration, but only those reasons, which are still left and not yet been shown.

A further possible step of the routine may be receiving a feedback from the user regarding fulfillment of the present unmet condition. Moreover, the invention relates to a system for tracking human motion with the features of claim 7. Accordingly, the system comprises:
- at least one sensor unit,
- at least one sensor holder;
   further comprising
   an analyzing module, configured and arranged such that it can be analyzed, whether there is a condition for correct sensor placement not met
   a presenting module, comprising a storage element, the storage element being equipped with potential reasons for the incorrect sensor placement, wherein the presenting module is further configured and arranged such that in case of detecting an unmet condition out of the storage element a list of potential reasons for the incorrect sensor placement is provided, and a selection module;
   wherein the presenting module is further capable to
   - Remove, by means of the selection module, the possible reasons for incorrect sensor placement already presented to a user;
   - Select, by means of the selection module, the one of the reasons and present it to a user.

Additionally, the storage element may be configured such that the reasons are stored within the storage element in a hierarchical ranking. Such a hierarchical ranking may be provided based on experience or test results and pre-stored in the storage element.

Furthermore, the hierarchical ranking may be also connected with a learning system and updated from time to time. Also such updates may be done automatically.

Furthermore, it is possible that the selection module may be configured such that it can be checked by means of the selection module, which reasons have already been presented to the user.

Additionally, it is possible that the selection module is configured such that it can be checked by means of the selection module, whether still reasons are left.

The presenting module can be further capable to wait for the user to acknowledge feedback.

In case that no reasons are left, the method routine can be ended by the system.

Additionally, it is possible that the selection module is configured such that if reasons are left, the hierarchically top ranked reason is selected and presented to the user by means of the presenting module.

Further details and advantages will be shown in the figures.

It is shown in
- Fig. 1: a perspective view of an embodiment of a system for tracking human motion according to the present invention;
- Fig. 2: a schematic overview of the communication between the sensor units and the main unit of the system according to Fig. 1;
- Fig. 3: a schematic view of the communication between the station and the main unit;
- Fig. 4: a flow chart of the sensor unit inspection procedures;
- Fig. 5: a person wherein the sensor units and the holders attached to the chest and to the upper arm and lower arm part;
- Fig. 6: a detailed view on the sensor holder and the sensor attached to the chest; and
- Fig. 7: an algorithm according to an embodiment of the present invention.

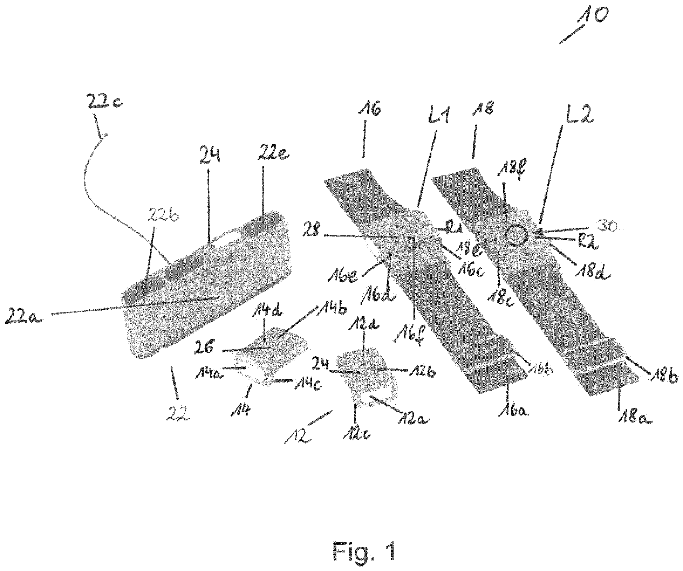

**Fig. 1** shows in a perspective view an embodiment of a system 10 for tracking human motion according to the present invention.

The system 10 comprises in the shown embodiment two sensor units 12 and 14.

Furthermore, there are sensor holders 16, 18, which are configured to receive and be attached with the sensor unit 12 or 14.

In the shown embodiment, sensor holders 16, 18 are configured and arranged to correspond and be attached during a work cycle/tracking cycle with sensor units 12, 14.

As long as sensor units 12, 14 are used, they can be received and placed in the sensor holders 16, 18, which form the respective counterelement.

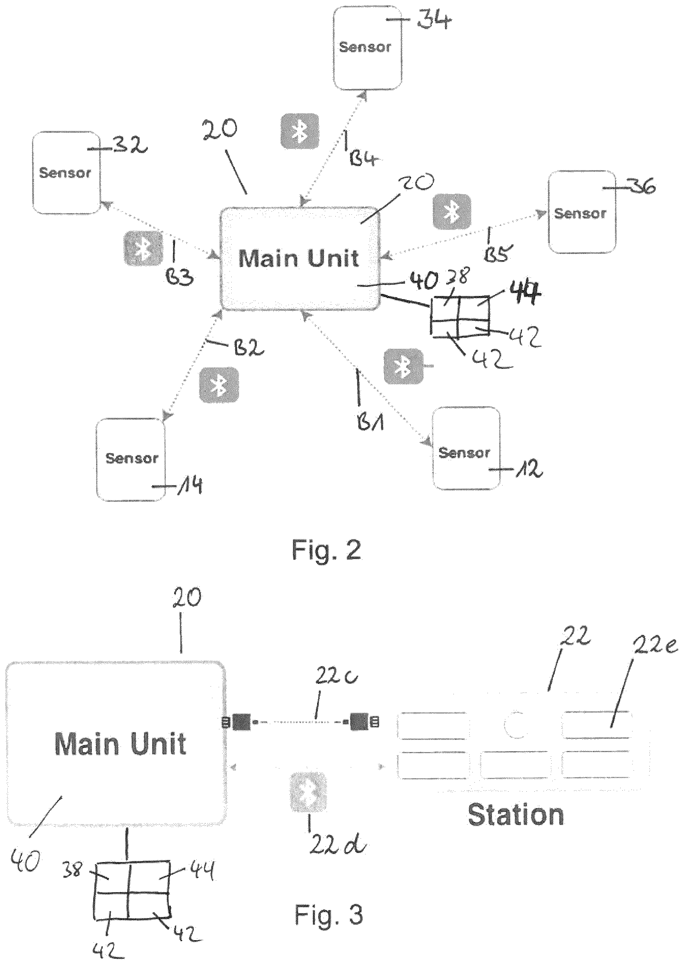

Moreover, the system 10 comprises a main unit 20, cf. also Fig. 2 and Fig. 3.

The main unit 20 is in the shown embodiment realized by a tablet PC.

Additionally, the system 10 comprises a sensor station 22.

The sensor station 22 comprises a plurality of receiving slots 22e, which are configured to receive the sensor units 12, 14.

The sensor station 22 is configured and arranged for re-charging of the sensor units 12, 14.

The sensor units 12, 14 comprise each a sensor communication interface 24, 26 and the sensor holders 16, 18 also comprise each a counter communication interface 28, 30.

The sensor units 12, 14 are identical. This applies in particular to the technical features and also to the design of the sensor units 12, 14 (and other sensor units).

Each sensor unit 12 or 14 has an L-form.

However, each sensor unit 12 or 14 is not personalized/individualized.

It is, however, possible that sensors can differ from each other and are personalized and/or individualized. Also, they can be named and/or provided with specific attachment information, where the sensor unit shall be placed.

The sensor unit 12, 14 has a color coding field 12a, 14a which has a color. This field can also be used for naming or branding.

Moreover, each sensor unit 12 or 14 has its own electronic identification circuit 12b or 14b.

With the electronic identification circuit 12b or 14b the sensor unit 12, 14 can identify a counterelement.

As counterelement for each electronic identification circuit 12b or 14b, there is an individualized resistor R1, R2 in each sensor holder 16, 18 as passive element.

Due to the fact that the sensor holders 16, 18 have individualized counterelements, it is not necessary that the sensor units are individualized.

To the contrary, in the shown embodiment the sensor units are completely identical as no strict correspondence between sensor units 12, 14 and sensor holders 16, 18 is needed.

Moreover, only the sensor holders 16, 18 comprise an identification of the limb, where they should be placed on and also an identification of the side of the body, to which they belong to and on which they should be placed.

The sensor holders 16, 18 are embodied such that they comprise wearable elastic straps 16a, 18a.

The wearable straps 16a, 18a comprise each at least one length adjustment element 16b, 18b, which is here a clamp 16b, 18b for length adjustment of the straps 16a, 18a.

With wearable straps 16a, 18a the sensor holders 16, 18 may be placed on the body or a limb of the patient.

On each elastic strap 16a, 18a a sensor unit attachment element 16c, 18c is provided, which serves as a docking element or docking station for one of sensor units 12, 14. Each sensor unit attachment element 16c, 18c has a form-fit or snap-fit for the sensor units 12, 14.

Here the form-fit and snap-fit is realized by means of elastic holding walls 16d, 18d and a recess 16e, 18e for the L-form protrusion 12c, 14c extending out of the main body 12d, 14d of the sensor unit 12 or 14.

Furthermore, there are snap-fit fingers 16f, 18f in the walls 16d, 18d, which are protruding out of the walls 16d, 18d and are arranged such that they realize a snap-fit contact with a sensor unit 12,14, once a sensor unit 12, 14 is inserted into the sensor unit attachment element 16c, 18c.

The sensor unit attachment elements 16c, 18c are attached to wearable straps 16a, 18a.

The sensor station 22 comprises a ON-OFF-Button 22a. This is, however, not mandatory.

Furthermore, the sensor station 22 comprises in each of its slots 22e an attachment communication interface 22b.

Also, there is a communication line 22c for connection with the main unit 20, which is here represented by a cable 22c.

Additionally, there is a Bluetooth link 22d as shown in Fig. 3.

Further (wireless) Bluetooth links B1, B2, B3, B4, B5 are established between each sensor unit 12, 14 and further sensor units 32, 34, 36 on the one side and the main unit 20 on the other side/in the center of the system 10.

All sensor units 12, 14, 32, 34, 36 are completely identical.

It would also be possible, to have one Bluetooth connection between one sensor master unit and the main unit and connect the other sensor units with the sensor master unit.

All sensors units 12, 14, 32, 34, 36 are each equipped with at least one inertial sensor.

Alternatively or additionally, as sensor may be used at least one of the sensor types gyroscope, acceleration sensor, piezo sensor, magnetic sensor, optical sensor or the like. In particular, the sensors/sensor units may comprise accelerometers and/or gyroscopes and/or magnetometers. Additionally, the system 10 comprises at least one analyzing module 38 and at least one presenting module 40 with at least one storage element 42.

The analyzing module 38 is configured and arranged such that it can be analyzed, whether there is a condition for correct sensor unit placement met or not met.

Furthermore, the storage element 42 of the presenting module 40 (e.g. the or a display of the main unit 20) is equipped with potential reasons for the incorrect sensor unit placement, wherein the presenting module 40 is further configured and arranged such that in case of detecting an unmet condition out of the storage element 42 a least of potential reasons for the incorrect sensor placement may be provided.

Furthermore, the system 10 comprises a selection module 44.

Furthermore, the presenting module 40 is capable to
- Remove by means of the selection module 44 the possible reasons for incorrect sensor unit placement already presented to a user;
- Select by means of the selection module 44 the one of the reasons and present it to a user;
- Wait for the user to acknowledge feedback.

The functionality can be described as follows:
The sensor units 12, 14, 32, 34, 36 are each configured such that depending on the correct placement of the sensor unit 12, 14, 32, 34, 36 to the sensor holder 16, 18 a communication link L1, L2 with the communication interface 24, 26 (of the sensor units) and the counter communication interface 28, 30 (of the sensor holder) is established.

Then, it can be inferred by the sensor unit 12, 14, whether it is attached to the sensor holder 16, 18 or not.

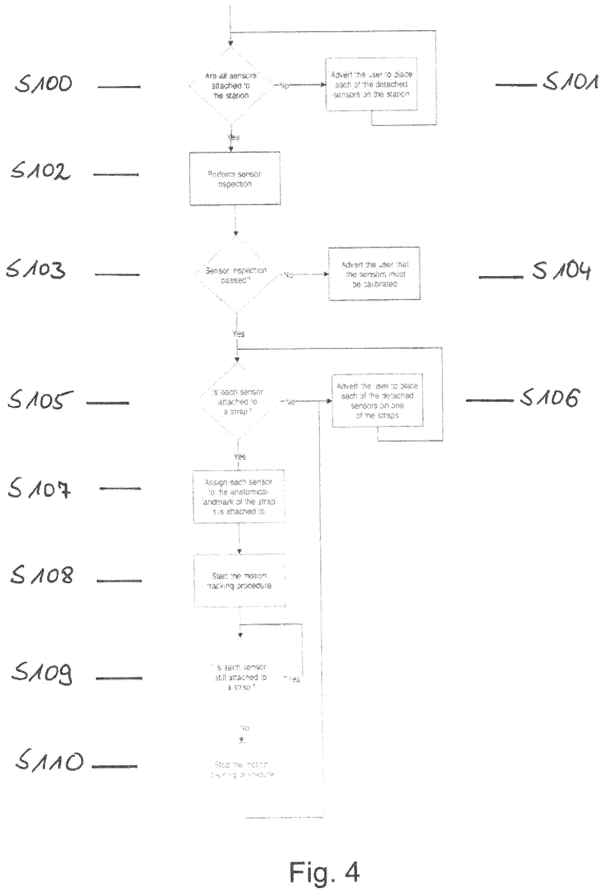

As described below in connection with **Fig. 4**, it is not necessary to have the sensor units and the sensor holders assigned to each other before the procedure.

The inspection procedure is a procedure where the sensor units are kind of "quality controlled" from the point of view that the measured values/signals provided by the sensor units are correct.

The main unit 20 is configured such that motion data as well as the information regarding the attachment of the sensor unit 12, 14 to the sensor holder 16, 18 can be tracked and/or processed.

The main unit 20 processes the motion data as well as the information regarding the attachment of each sensor unit 12, 14, 32, 34, 36 and provides feedback to the user via the display of a tablet device, here forming the main unit 20.

Sensor units 12, 14, 32, 34, 36 (also called motion sensors) are placed in anatomical landmarks, they collect the user's motion data as well as information regarding which component they are attached to and send it to the main unit 20 through a wireless communication protocol (here the sensor units 12, 14, 32, 34, 36 are embodied as devices comprising a microcontroller, Bluetooth connectivity, inertial sensors and the attachment communication interface).

The sensor holders 16, 18 with the wearable elastic straps 16a, 18a securely attach each sensor unit 12, 14, 32, 34, 36 to the corresponding anatomical landmark (of a patient).

Each sensor holder 16, 18 is equipped with a counter communication interface 28, 30 (also called attachment communication interface 28, 30).

The sensor station 22 holds the sensor units 12, 14, 32, 34, 36 for charging and inspection purposes. Equipped with the attachment communication interface 22b, this component communicates with the main unit 20 through the cable/ communication line 22c and/or the wireless communication protocol, here the additional Bluetooth link 22d.

This interface, i.e. the attachment communication interface 22b of the sensor station 22 or the counter communication interface 28, 30 of the sensor holder 16, 18 provides the advantage that the sensor units 12, 14, 32, 34, 36 have the capability of inferring to which piece of hardware they are connect (if any).

In its simplest form, it can be embodied as a couple of terminals connected by a resistor on the sensor holder/sensor station side and an active circuit on the sensor unit side. With such configuration and by having a different resistor value for each of the straps as well as the sensor station, the microcontroller in the sensor is able to measure resistance between its terminals continuously and thus infer whether it is disconnected, connected to one of the straps or to the sensor station. By having a passive circuit on the sensor holder side, one can avoid the need of charging the straps which is obviously of interest for the sake of the user experience.

Each of the sensor holders 16, 18, as well as the sensor station 22, is equipped with a hardware interface that connects to a homologous interface in the sensors upon their attachment. This interface and the connection provide each individual sensor unit with the capability of inferring whether it is attached to a specific strap, attached to the base station or detached. As a result the system is able to:
- Make sure the sensor units are correctly attached to the sensor holders and advert the user if they are not before the motion tracking procedure begins.
- Advert the user when a sensor detaches from its strap during the motion tracking procedure.
- Make sure the sensor inspection procedure is valid by requiring the user to have the sensors correctly attached to the station.
- Ensure a proper charging procedure of the sensors by requiring the user to have the sensors correctly attached to the station.
- Allow the user to attach each sensor to a random strap since the system is now capable of performing the match between each sensor and the strap without having to rely on a predefined configuration the user would have to comply with (as is the case of the color codes).

These features have an advantageous and beneficial impact on the user experience as the system is now able to guide the user through each step by providing relevant and adaptive feedback whilst ensuring proper functioning.

Generally speaking the method of using the system 10 has at least the following steps:
- the sensor unit(s) 12, 14, 32, 34, 36 is/are placed in the sensor holder(s) 16, 18;
- a communication link L1, L2 between the sensor unit 12, 14 and the sensor holder 16, 18 is established; and
- based on the communication link L1, L2 and the information exchanged via this communication link L1, L2 it is inferred by the sensor unit 12, 14, whether it is attached to the sensor holder 16, 18 or not.

With the established communication link L1, L2 an individualized element of the sensor holder 16, 18, here the resistors R1, R2, is/are checked and identified by means of the sensor unit 12, 14, 32, 34, 36.

Further details are explained in connection with **Fig. 4****.**

**Fig. 4** shows a flow chart of the system setup procedure (also including a sensor unit inspection routine).

This routine comprises the necessary steps a user must perform in the shown embodiment of the system to start tracking its motion with the system 10. Similar setups and routines are generally possible.

Here, at least one or more or all of the sensor units 12, 14, 32, 34, 36 is/are attached to the sensor station 22, i.e. the at least one sensor unit 12, 14, 32, 34, 36 is put in one of the slots 22e.

In step S100 it is checked, whether all sensor units 12, 14, 32, 34, 36 (here all, it might in an alternative embodiment be also possible that for the check only one or several sensor units or all sensor units must be placed into the sensor station 22) are attached to the sensor station 22.

If this is not the case, the procedure continues in step S101, i.e. the user is invited to place each of the detached sensors into the slots 22e of the sensor station 22. Then, it is continued with step S100 again.

If all sensor units 12, 14, 32, 34, 36 are then attached to the sensor station 22, then in step S102 the sensor unit inspection is performed.

After the sensor inspection of step S102 a decision is taken in step S103, whether the sensor inspection is passed or not.

If the sensor inspection is not passed, then in step S104 the user is invited to calibrate the sensor units 12, 14, 32, 34, 36.

If the sensor inspection is passed, then in step S105 it is checked, whether each sensor unit 12, 14, 32, 34, 36 is attached to one sensor holder 16, 18.

This is checked as described above by means of the sensor communication interface 24, 26 and the passive counter communication interface 28, 30 of the sensor holder 16, 18.

If the check of step S105 reveals that not all sensor units 12, 14, 32, 34, 36 are correctly placed, the user is invited in step S106 to place each of the detached sensors units 12, 14, 32, 34, 36 to one sensor holder, 16, 18 (one sensor unit to one sensor holder).

Steps S101 ("Advert the user to place each of the detached sensors on the station"), S104 ("Advert the user that the sensors must be calibrated") and S106 ("Advert the user to place each of the detached sensors on one of the straps") can involve the step of displaying such a message by means of the display on the main unit 20.

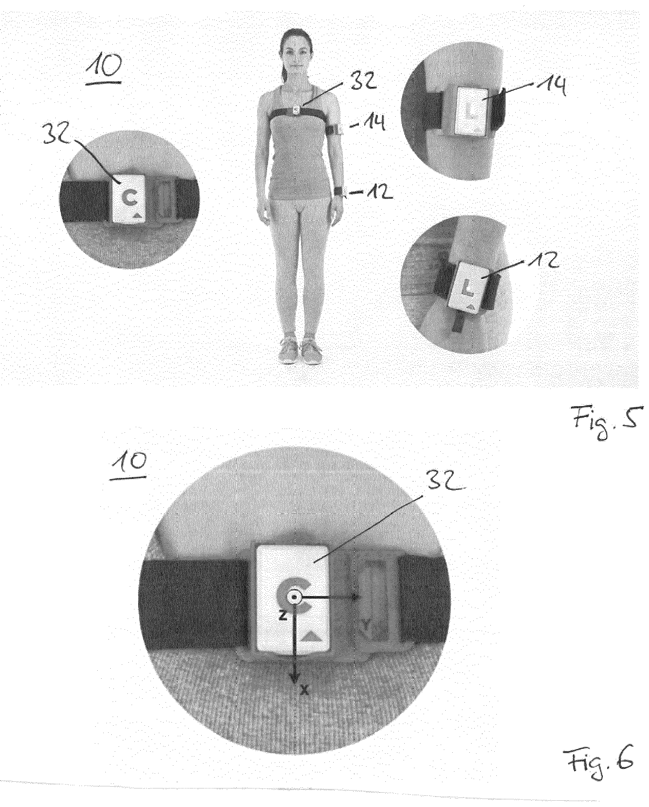

The detailed sensor placement error detection and correction procedure of the system 10 is described below in connection with **Fig. 5-7****.**

If the system 10 comes in check S105 to the result that each sensor is attached to a sensor holder 16, 18, then in step S107 each sensor unit 12, 14, 32, 34, 36 is assigned to the anatomical landmark of the sensor holder 16, 18 it is attached to. After that, in step S108 the motion tracking procedure is started.

During this procedure, there is a check routine in step S109, which is following step S108. Here, it is continuously (or alternatively intermittently) checked, whether each sensor unit 12, 14, 32, 34, 36 is still attached to a sensor holder 16, 18.

In step S110, the motion tracking procedure is stopped, when in step S109 it is found that one or more of the sensor units 12, 14, 32, 34, 36 are no longer attached to the respective sensor holder 16, 18.

If so, it is switched back to step S106.

Then the procedure may continue from there on.

The procedure for sensor placement error detection and correction works as follows:
The problem to be solved has to do with the fact that the system 10, which is capable of tracking human motion, requires the user to place a set of sensor units on anatomical landmarks as described above. depending upon the limb whose motion is to be tracked.

Due to these requirements and the fact that it is not clear for the user what he must do at a given point while using the system 10, the following problems arise:
- The user does not place the sensor units on the body because he does not understand he must do so; and/or
- The user misplaces the sensor units by switching positions between two sensor units since this match is cumbersome and prone to failure; and/or
- The user does not switch positions between sensor units but he does not place one or more sensor units on the required anatomical landmarks.

These events result in a corruption of the motion tracking procedure.

In order to overcome this issue, the method and also the system rely on an algorithm, which assesses the orientations reported by the sensor units at a step before the actual motion tracking procedure where the patient is asked to stand still in a specified position according to the exercises he will perform (e.g. standing upright, seated upright, lying on his back, etc). At that point these orientations are analysed and a set of metrics is computed. Each of these metrics is then compared against an allowable interval (which might be open on one of the limits) and the placement is considered valid if the value each metric falls within the respective interval. These intervals/metrics are essentially a set of conditions that the reported orientations meet, in either an absolute or a relative manner, when the sensors are correctly placed.

This is e.g. based on predefined conditions regarding angles of the sensor units (e.g. to each other or within the threedimensional space). There can be also predefined conditions based on angles and predefined intervals, in which specific conditions must be met.

Each condition can be e.g. defined as a logical AND or a logical OR of one or more intervals of angles and allowable intervals.

Each of these conditions is associated with one or more possible causes of its violation. All causes of violation (even those regarding different conditions) have a hierarchical relation between them. This hierarchy expresses the order through which the causes of violation will presented to the patient and is a result of the likelihood of the cause as well as the fact that some errors must be corrected before other errors may be detected (e.g. a patient must first fix an error related to switching a couple of sensor units before an error related to a misplacement of a single sensor unit may be detected).

When one or more causes of violation are eligible - because the violated condition has more than one possible cause and/or because more than one condition was violated - the system selects the hierarchically superior cause and presents it to the patient. Then, the system expects the patient to check whether he may have misplaced the sensor units as indicated by the presented cause and, if so, act accordingly.

When he is done he interacts with the system 10 to signal another analysis may be triggered. At this point the system gathers all the possible causes of violation once more but this time removes the cause that has already been presented to the patient (if it is still violated) before selecting the next cause of violation to be presented. This process may continue recursively until all the conditions are satisfied or until a specified number of attempts is performed.

For the purpose of illustrating the disclosure an example will be provided for the case of a sensor setup comprising on sensor unit 12 on the left lower arm, one sensor unit 14 on the lateral side of the left upper arm and one sensor unit 32 on the chest, as shown in **Fig. 5****.**

To that end, it will be assumed that the axes of reference of each a sensor unit are those illustrated in **Fig. 6** and that all the conditions will be expressed as angles between two axes of reference of different sensor units or between an axis of reference and a global vector. In regards to the global reference frame, hereinafter will be assumed that x points towards the north, z points up and y is given by their cross product.

The list of conditions that must be satisfied in order for the placement to be considered valid are presented in **Table 1**. As an example, the first condition is that the angle between the (chest) red sensor's x vector and the global z vector (the 'up' vector) must be lower than 90o. At the step in which the patient is asked to stand still in an upright position the system asserts the validity of each of these conditions and if any of them is invalid the sensor placement is deemed incorrect.

**Tbl. 1**

| **Condition** | **Assertion** |
|---|---|
| 1 | *angle*(x_{RED,} z_{GLOBAL}) < 90° |
| | |
| 2 | *angle*(x_{GREEN,} z_{GLOBAL}) < 90° |
| 3 | *angle*(x_{GREEN,} z_{GLOBAL}) < 45° and *angle*(x_{GREEN,} z_{GLOBAL}) > 135° or *angle*(x_{BLUE,} z_{GLOBAL}) ∈ [45°; 135°] |
| 4 | *angle*(y_{RED,} z_{GREEN}) < 45° |

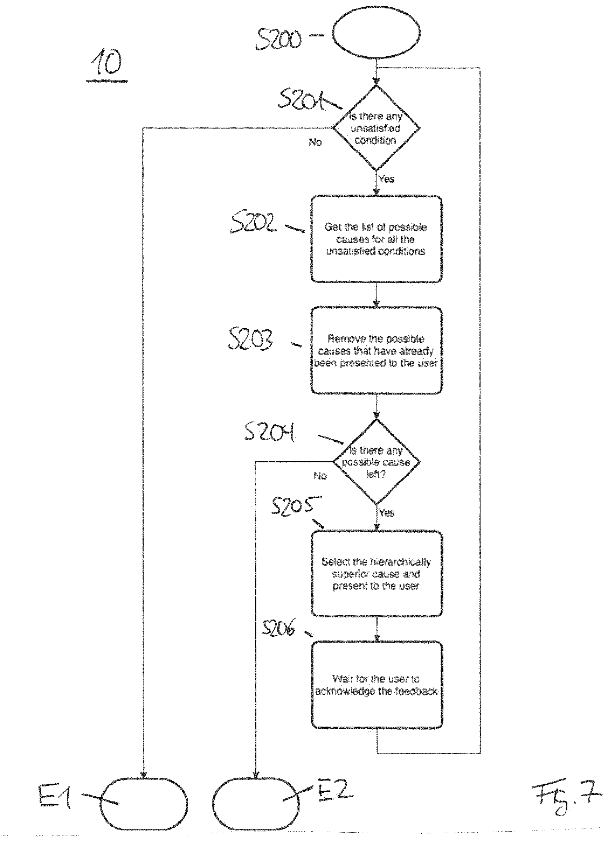

The provision of feedback is accomplished by the algorithm described in **Fig. 7****.** As one can see, the algorithm has two possible ending scenarios E1 and E2, the first is when at some point all the conditions are satisfied and the second is when even after being presented with all the possible error causes the user still does not manage to place the sensors correctly. There could also be another scenario where the number of possible causes presented to the user is limited and the algorithm ends when that number is reached.

In detail, the following steps are performed according to one embodiment of the method of the present invention, performed on a system 10 according to the present invention:
In Step S200, the algorithm is started.
In Step S201, it is checked by means of the analyzing module 38, whether there is any unsatisfied condition.

If this is not the case, the endpoint E1 is reached (i.e. ending scenario E1). Then, there is no problem at all regarding unsatisfied conditions in connection with sensor placement.

If there is an unsatisfied condition detected by means of the analyzing module, then the list of possible causes is provided for all the unsatisfied conditions out of the storage element 42 of the presenting module 40 in step S202.

The list of conditions along with the respective possible causes is exemplarily shown in **Table 2** below:

**Tbl. 2**

| **Condition** | **Possible Cause(s) of Violation** | |
|---|---|---|
| | **Description** | **Hierarchy Level** |
| 1 | *The red sensor is upside down* | 3 |
| | | |
| 2 | *The green sensor is upside down* | 2 |
| 3 | *The blue and green sensors are switched* | 1 |
| 4 | *The red sensor is placed on the back rather than on the chest* | 2 |
| | *The green sensor is on the right upper arm* | 1 |
| | *The green sensor is not on the outer side of the left arm* | 2 |

In step S203 the possible causes are removed that have already been presented to the user by the selection module 44.

After that, in step S204 it is checked, whether there is any possible cause left.

If this is not the case, then the endpoint E2 (i.e. ending scenario E2) is reached. Then, there is no longer a problem at all regarding unsatisfied conditions in connection with sensor placement or the reason cannot be detected this way.

If there is still a possible cause left, then it is continued in step S204.

In step S205, the hierarchically superior cause (i.e. the cause, which is among the left over ones and which is most prominently ranked) is chosen by the selection module 44 and presented to the user by means of the presenting module 40.

After that in step S206 it is waited for the user input to acknowledge the feedback and it is then jumped back to step S201 .

Note that the example control and estimation routines included herein can be used with various system configurations. The control methods and routines disclosed herein may be stored as executable instructions in non-transitory memory and may be carried out by a control unit e.g. as a part of the main unit 20 in combination with the various sensors, actuators, and other system hardware. The specific routines described herein may represent one or more of any number of processing strategies such as event-driven, interrupt-driven, multi-tasking, multi-threading, and the like. As such, various actions, operations, and/or functions illustrated may be performed in the sequence illustrated, in parallel, or in some cases omitted. Likewise, the order of processing is not necessarily required to achieve the features and advantages of the example embodiments described herein, but is provided for ease of illustration and description. One or more of the illustrated actions, operations and/or functions may be repeatedly performed depending on the particular strategy being used. Further, the described actions, operations and/or functions may graphically represent code to be programmed into non-transitory memory of the computer readable storage medium in the control unit, where the described actions are carried out by executing the instructions in a system including the various hardware components in combination with the electronic control unit.

### References

- 10: system for tracking human motion
- 12: sensor unit
- 12a: color coding field
- 12b: electronic identification circuit
- 12c: L-form protrusion
- 12d: main body
- 14: sensor unit
- 14a: color coding field
- 14b: electronic identification circuit
- 14c: L-form protrusion
- 14d: main body
- 16: sensor holder
- 16a: strap
- 16b: clamp
- 16c: sensor unit attachment element
- 16d: wall
- 16e: recess
- 16f: snap fit finger
- 18: sensor holder
- 18a: strap
- 18b: clamp
- 18c: sensor unit attachment element
- 18d: wall
- 18e: recess
- 18f: snap fit finger
- 20: main unit
- 22: sensor station
- 22a: ON-OFF-Button
- 22b: attachment communication interface
- 22c: communication line
- 22d: Bluetooth link
- 22e: slot
- 24: sensor communication interface
- 26: sensor communication interface
- 28: counter communication interface
- 30: counter communication interface
- 32: sensor unit
- 34: sensor unit
- 36: sensor unit
- 38: analyzing module
- 40: presenting module
- 42: storage element
- 44: selection module

- L1: communication link
- L2: communication link

- S100: step of sensor unit inspection routine
- S101: step of sensor unit inspection routine
- S102: step of sensor unit inspection routine
- S103: step of sensor unit inspection routine
- S104: step of sensor unit inspection routine
- S105: step of sensor unit inspection routine
- S106: step of sensor unit inspection routine
- 5107: step of sensor unit inspection routine
- S108: step of sensor unit inspection routine
- S109: step of sensor unit inspection routine
- S110: step of sensor unit inspection routine

- S200: step of sensor unit placement error detection and correction routine
- S201: step of sensor unit placement error detection and correction routine
- S202: step of sensor unit placement error detection and correction routine
- S203: step of sensor unit placement error detection and correction routine
- S204: step of sensor unit placement error detection and correction routine
- S205: step of sensor unit placement error detection and correction routine
- S206: step of sensor unit placement error detection and correction routine

- E1: endpoint 1 / ending scenario 1
- E2: endpoint 2 / ending scenario 2

- R1: resistor
- R2: resistor

## Claims

1. A method of performing sensor placement error detection and correction for a system (10) for tracking human motion comprising
- at least one sensor unit (12,14),
- at least one sensor holder (16,18),
wherein the method comprises at least the following steps forming a method routine:
- Analyzing, whether there is at least one unmet condition, indicating an incorrect sensor unit placement;
- In case of detecting such at least one unmet condition preparing a list of potential reasons for the incorrect sensor unit placement;
- Remove the possible reasons for incorrect sensor unit placement already presented to the user;
- Select the one of the reasons and present it to a user.

2. The method according to claim 1, **characterized in that** the reasons are hierarchically ranked.

3. The method according to claim 1 or 2, **characterized in that** it is checked, which reasons have already been presented.

4. The method according to one of the preceding claims, **characterized in that** it is checked, whether still reasons are left.

5. The method according to claim 4, **characterized in that** in case that no reasons are left, the method routine is ended.

6. The method according to claim 4, **characterized in that** in case that if reasons are left, the hierarchically top ranked reason is selected and presented to the user.

7. The method according to one of the preceding claims, **characterized in that** a feedback from the user regarding fulfillment of the present unmet condition is received.

8. A system (10) for tracking human motion comprising:
- at least one sensor unit (12,14),
- at least one sensor holder (16,18),
further comprising
an analyzing module (38), configured and arranged such that it can be analyzed, whether there is a condition for correct sensor unit placement not met,
a presenting module (40), comprising a storage element (42), the storage element (42) being equipped with potential reasons for the incorrect sensor unit placement, wherein the presenting module (40) is further configured and arranged such that in case of detecting an unmet condition out of the storage element (42) a least of potential reasons for the incorrect sensor unit placement is provided, and a selection module (44);
wherein the presenting module (40) is further capable to
- Remove, by means of the selection module (44), the possible reasons for incorrect sensor placement already presented to a user;
- Select, by means of the selection module (44), the one of the reasons and present it to a user.

9. The system (10) according to claim 8, **characterized in that** the storage element (42) is configured such that the reasons are stored within the storage element (42) in a hierarchical ranking.

10. The system (10) according to claim 8 or claim 9, **characterized in that** the selection module (42) is configured such that it can be checked by means of the selection module (42), which reasons have already been presented.

11. The system (10) according to one of claims 8 to 10, **characterized in that** the selection module (42) is configured such that it can be checked by means of the selection module (42), whether still reasons are left.

12. The system (10) according to claim 11, **characterized in that** the selection module (42) is configured such that if no reasons left, the method routine is ended.

13. The system (10) according to claim 12, **characterized in that** the selection module (44) is configured such that if reasons are left, the hierarchically top ranked reason is selected and presented to the user by means of the presenting module (40).

14. The system (10) according to one of claims 8 to 13, **characterized in that** the presenting module (40) is further capable to wait for the user to acknowledge feedback.
